# EUROPEAN PATENT APPLICATION

(11) **EP 4 591 926 A1**
(43) Date of publication of application: **30.07.2025**
(21) Application number: 24845301.1
(22) Date of filing: 26.06.2024
(51) Int. Cl.: A61N 7/00, A61B 8/14, A61N 7/02

(54) **HIFU IRRADIATION DEVICE AND FOCUS ADJUSTMENT METHOD**

(30) Priority: 21.07.2023 JP 2023118823; 23.04.2024 JP 2024069710
(71) Applicant: SONIRE THERAPEUTICS INC., Tokyo 103-0023 (JP)
(72) Inventor: UEYAMA, Tsuyoshi, Tokyo 103-0023 (JP); URAYAMA, Hiroaki, Tokyo 103-0023 (JP); SATOH, Tohru, Tokyo 103-0023 (JP)
(74) Representative: Novagraaf International SA
(86) International application number: PCT/JP2024/023185
(87) International publication number: WO 2025/022919

(57) **Abstract**

The purpose of the present invention is to facilitate, in an HIFU irradiation device, an operation for aligning the position of the focal point of therapeutic ultrasound with a treatment site. A vibrator control unit (18) controls a plurality of ultrasonic vibrators (32) disposed along a concave surface (30). A probe drive unit (14) causes an ultrasonic probe (12), which is disposed on the central axis (40) of the concave surface (30), to rotate around the central axis (40). A data acquisition unit (16) acquires B-mode image data by means of the ultrasonic probe (12). A controller (22) controls the vibrator control unit (18), the probe drive unit (14), and the data acquisition unit (16). When the plurality of ultrasonic vibrators (32) are being caused to transmit ultrasound, the controller 22 executes a process for searching for the focal point F of the ultrasound being emitted from the plurality of ultrasonic vibrators (32) on the B-mode image represented by the B-mode image data while changing the rotation angle position of the ultrasonic probe (12) around the central axis (40).

## Description

### TECHNICAL FIELD

The present invention relates to a HIFU sonication apparatus and a method of adjusting a focal point thereof, and in particular to adjustment of a position of the focal point using a B mode image.

### BACKGROUND

A therapeutic apparatus which uses high intensity focused ultrasound (HIFU) is in wide use. The therapeutic apparatus is called a HIFU sonication apparatus or a HIFU sonication system, and illuminates focused ultrasound on a therapeutic site, to necrotize biological tissues.

In general, the HIFU sonication apparatus comprises a plurality of ultrasound transducers for therapy, which are placed along a concave surface. The plurality of ultrasound transducers are placed in such a manner that ultrasounds emitting therefrom are illuminated onto one point, and form a focal point. During therapy, the position of the focal point is set at the therapeutic site, and the ultrasounds are illuminated. In order to check the sonication position, an ultrasound diagnostic apparatus which indicates the focal point on an ultrasound image is used.

Patent Document 1 discloses an ultrasound therapeutic apparatus which observes the position of the focal point using an ultrasound diagnostic apparatus which displays a B mode image (tomographic image). In this apparatus, ultrasound of a weak level which does not affect the tissues is emitted from an ultrasound transducer for therapy, and a tomographic image is displayed through transmission and reception of ultrasound by an ultrasound imaging probe. Because acoustic characteristics of the tissue of a patient vary depending on a temperature change of the tissue, the position of the focal point is shown in the tomographic image as a magnitude of brightness. In addition, as shown in Non-Patent Document 1, because a displacement is caused in the tissue of the patient due to the sonication of the ultrasound, the position of the focal point is shown in the tomographic image as a change of the brightness.

### CITATION LIST

### PATENT LITERATURE

Patent Document 1: JP H8-71069 A

### NON PATENT LITERATURE

Non-Patent Document 1: Tissue Hardness Measurement using ARFI, on the Internet, <https://www.innervision.co.jp/sp/ad/suite/siemens/technical_notes/140368>

### SUMMARY

### TECHNICAL PROBLEM

In therapy using the high intensity focused ultrasound, if a region with acoustic characteristics different from the surroundings exists in the tissue of the patient, there may be a case where, due to refraction of the ultrasound emitted from the ultrasound transducer for therapy, the position of the focal point is deviated from that in the case of uniform acoustic characteristics. As a consequence, there may be a case where the focal point of the ultrasound does not appear on the B mode image, and manipulation for setting the position of the focal point of the therapeutic ultrasound at the therapeutic site becomes difficult.

An advantage of the present invention lies in facilitating the manipulation to set the position of the focal point of the therapeutic ultrasound at the therapeutic site in the HIFU sonication apparatus.

### SOLUTION TO PROBLEM

According to one aspect of the present invention, there is provided a HIFU sonication apparatus comprising: a transducer control unit configured to control a plurality of ultrasound transducers placed along a concave surface; a probe driver that rotates an ultrasound probe, placed on a central axis of the concave surface, about the central axis; a data acquisition unit that acquires B mode image data by the ultrasound probe; and a controller configured to control the transducer control unit, the probe driver, and the data acquisition unit, wherein the controller is configured to execute: a process in which, during a period in which the plurality of ultrasound transducers are caused to transmit ultrasound, while a rotational angle position of the ultrasound probe about the central axis is changed, a focal point, of the ultrasounds emitted from the plurality of ultrasound transducers and which appears as a magnitude of brightness in a B mode image indicated by the B mode image data, is searched for based on the B mode image data; and a process in which the ultrasound probe is caused to rest at the rotational angle position at which the focal point is detected.

Desirably, the controller is configured to, in a state in which the ultrasound probe rests at the rotational angle position at which the focal point is detected, adjust a delay time of the ultrasound emitted from each of the plurality of ultrasound transducers, so that the focal point moves closer to the central axis or the focal point is positioned on the central axis.

According to another aspect of the present invention, there is provided a HIFU sonication apparatus comprising: a transducer control unit configured to control a plurality of ultrasound transducers placed along a concave surface; an ultrasound transducer driver that rotates the plurality of ultrasound transducers about a central axis of the concave surface; a data acquisition unit that acquires B mode image data by an ultrasound probe placed on the central axis of the concave surface; and a controller configured to control the transducer control unit, the ultrasound transducer driver, and the data acquisition unit, wherein the controller is configured to execute: a process in which, during a period in which the plurality of ultrasound transducers are caused to transmit ultrasounds, while rotational angle positions of the plurality of ultrasound transducers about the central axis are changed, a focal point, of the ultrasounds emitted from the plurality of ultrasound transducers and which appears as a magnitude of brightness in a B mode image indicated by the B mode image data, is searched for based on the B mode image data; and a process in which the plurality of ultrasound transducers are caused to rest at the rotational angle positions at which the focal point is detected.

Desirably, the controller is configured to, in a state in which the plurality of ultrasound transducers rest at the rotational angle positions at which the focal point is detected, adjust a delay time of the ultrasound emitted from each of the plurality of ultrasound transducers, so that the focal point moves closer to the central axis or the focal point is positioned on the central axis.

According to another aspect of the present invention, there is provided a method of adjusting a focal point of a HIFU sonication apparatus, wherein the HIFU sonication apparatus comprises: a plurality of ultrasound transducers placed along a concave surface; an ultrasound probe placed on a central axis of the concave surface, in a manner to be rotatable about the central axis; and a data acquisition unit which acquires B mode image data by the ultrasound probe, the HIFU sonication apparatus further comprises: a transducer control unit configured to control the plurality of ultrasound transducers; and a probe driver which rotates the ultrasound probe about the central axis, and a controller, configured to control the transducer control unit, the probe driver, and the data acquisition unit, is configured to execute: a step in which, during a period in which the plurality of ultrasound transducers are caused to transmit ultrasounds, while a rotational angle position of the ultrasound probe about the central axis is changed, a focal point, of the ultrasounds emitted from the plurality of ultrasound transducers and which appears as a magnitude of brightness in a B mode image indicated by the B mode image data, is searched for based on the B mode image data; and a step in which the ultrasound probe is caused to rest at the rotational angle position at which the focal point is detected.

Desirably, the method includes a step in which, in a state in which the ultrasound probe rests at the rotational angle position at which the focal point is detected, a delay time of each of the ultrasounds emitted from the plurality of ultrasound transducers is adjusted, so that the focal point moves closer to the central axis or the focal point is positioned on the central axis.

According to another aspect of the present invention, there is provided a method of adjusting a focal point of a HIFU sonication apparatus, wherein the HIFU sonication apparatus comprises: a plurality of ultrasound transducers placed along a concave surface, and placed in a manner to be rotatable about a central axis of the concave surface; an ultrasound probe placed on the central axis of the concave surface; and a data acquisition unit which acquires B mode image data by the ultrasound probe, the HIFU sonication apparatus further comprises: a transducer control unit configured to control the plurality of ultrasound transducers; and a probe driver which rotates the ultrasound probe about the central axis; and a controller, configured to control the transducer control unit, the probe driver, and the data acquisition unit, is configured to execute: a step in which, during a period in which the plurality of ultrasound transducers are caused to transmit ultrasounds, while rotational angle positions of the plurality of ultrasound transducers about the central axis are changed, a focal point, of the ultrasounds emitted from the plurality of ultrasound transducers and which appears as a magnitude of brightness in a B mode image indicated by the B mode image data, is searched for based on the B mode image data; and a step in which the plurality of ultrasound transducers are caused to rest at the rotational angle positions at which the focal point is detected.

Desirably, the method includes a step in which, in a state in which the plurality of ultrasound transducers rest at the rotational angle positions at which the focal point is detected, a delay time of each of the ultrasounds emitted from the plurality of ultrasound transducers is adjusted, so that the focal point moves closer to the central axis or the focal point is positioned on the central axis.

According to another aspect of the present invention, there is provided a HIFU sonication apparatus comprising: a transducer control unit configured to control a plurality of ultrasound transducers placed along a concave surface; a data acquisition unit that acquires B mode image data by an ultrasound probe placed on a central axis of the concave surface; and a controller configured to control the transducer control unit and the data acquisition unit, wherein the controller is configured to execute a process in which, during a period in which the plurality of ultrasound transducers are caused to transmit ultrasounds, while a delay time of the ultrasound emitted from each of the plurality of ultrasound transducers is adjusted, a focal point, of the ultrasounds emitted from the plurality of ultrasound transducers and which appears as a magnitude of brightness in a B mode image indicated by the B mode image data, is searched for based on the B mode image data. The controller may be configured to execute a process in which, during a period in which the plurality of ultrasound transducers are caused to transmit the ultrasounds, while a delay time of the ultrasound emitted from each of the plurality of ultrasound transducers is adjusted, a focal point of the ultrasounds emitted from the plurality of ultrasound transducers is searched for in a B mode image indicated by the B mode image data.

Desirably, in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.

According to another aspect of the present invention, there is provided a method of adjusting a focal point of a HIFU sonication apparatus, wherein the HIFU sonication apparatus comprises: a plurality of ultrasound transducers placed along a concave surface; and a data acquisition unit which acquires B mode image data by an ultrasound probe placed on a central axis of the concave surface, the HIFU sonication apparatus further comprises a transducer control unit configured to control the plurality of ultrasound transducers, and a controller, configured to control the transducer control unit and the data acquisition unit, is configured to execute a step in which, during a period in which the plurality of ultrasound transducers are caused to transmit ultrasounds, while a delay time of the ultrasound emitted from each of the plurality of ultrasound transducers is adjusted, a focal point, of the ultrasounds emitted from the plurality of ultrasound transducers and which appears as a magnitude of brightness in a B mode image indicated by the B mode image data, is searched for in a B mode image indicated by the B mode image data based on the B mode image data, the focal point being a focal point of the ultrasounds emitted from the plurality of ultrasound transducers. The method of adjusting the focal point may further include a step in which, during a period in which the plurality of ultrasound transducers are caused to transmit ultrasounds, while a delay time of the ultrasound emitted from each of the plurality of ultrasound transducers is adjusted, a focal point of the ultrasounds emitted from the plurality of ultrasound transducers is searched for in a B mode image indicated by the B mode image data.

Desirably, the method further includes a step in which, in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.

### ADVANTAGEOUS EFFECTS OF INVENTION

According to the present invention, the manipulation to set the position of the focal point of the therapeutic ultrasound at the therapeutic site can be facilitated.

### BRIEF DESCRIPTION OF DRAWINGS

FIG. 1 is a diagram showing a structure of a HIFU sonication apparatus according to a first embodiment of the present invention.
FIG. 2 is a diagram for explaining a focal point adjustment process.
FIG. 3 is a diagram for explaining a focal point adjustment process.
FIG. 4 is a diagram for explaining a focal point adjustment process.
FIG. 5 is a diagram showing a structure of a HIFU sonication apparatus according to a second embodiment of the present invention.

### DESCRIPTION OF EMBODIMENTS

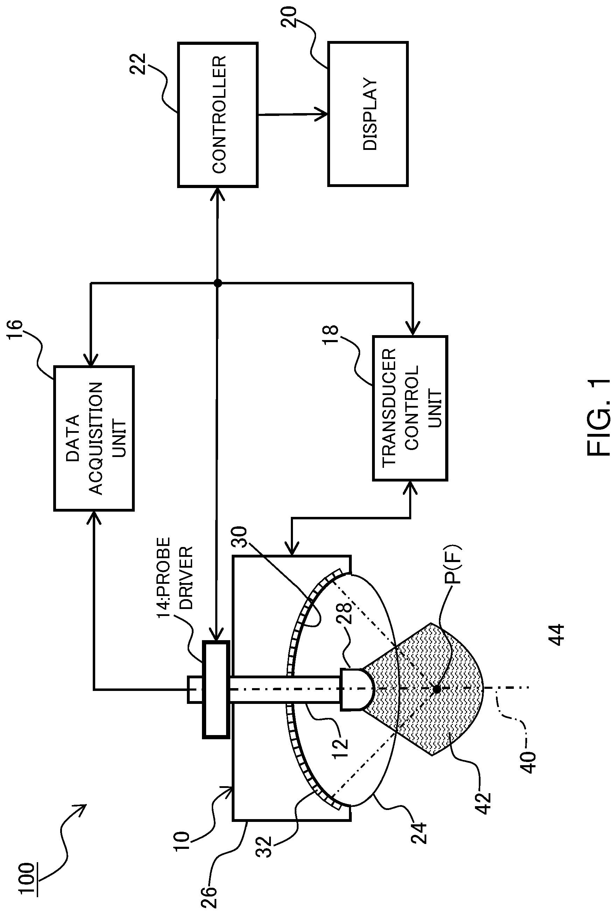

Embodiments of the present invention will now be described with reference to the drawings. Identical constituent elements shown on different drawings are assigned the same reference numerals, and descriptions thereof will not be repeated. FIG. 1 shows a structure of a HIFU sonication apparatus 100 according to a first embodiment of the present invention. The HIFU sonication apparatus 100 comprises a HIFU transducer unit 10, an ultrasound probe 12, a coupling bag 24, a probe driver 14, a data acquisition unit 16, a transducer control unit 18, a display 20, and a controller 22.

FIG. 1 shows a cross section of the HIFU transducer unit 10. The HIFU transducer unit 10 has a transducer housing 26 having a concave surface 30 with an opening facing downward, and a plurality of ultrasound transducers 32 placed along the concave surface 30 of the transducer housing 26 and fixed on the transducer housing 26. The HIFU transducer unit 10 does not necessarily have a real concave surface 30. In this case, the plurality of ultrasound transducers 32 may be fixed on the transducer housing 26 so as to be placed along a virtual concave surface.

The concave surface 30 of the transducer housing 26 may have a shape similar to a side surface of a cone. Here, a "cone" refers to a three-dimensional shape formed by a collection of straight lines extending from one point in a space toward a bottom surface. Further, the concave surface 30 of the transducer housing 26 may have a shape swollen on the upper side in a dome shape. The ultrasound transducers 32 are fixed on the transducer housing 26 in such a manner that, when the ultrasound transducers 32 emit ultrasound, intensity of the ultrasounds is strengthened at a therapeutic reference point P below the transducer housing 26.

The controller 22 may be a personal computer, a tablet computer, or the like. The controller 22 may execute various functions through execution of a program. To the controller 22, a manipulation device (not shown) for a user to manipulate the HIFU sonication apparatus 100 is connected. The manipulation device may include a mouse, a touch panel integrated with the display 20, a switch, a keyboard, or the like.

The transducer control unit 18 may be formed from one or more processors configured to control the ultrasound transducers 32 through execution of a program. The transducer control unit 18 causes the ultrasound transducers 32 to generate ultrasounds in response to control of the controller 22. The transducer control unit 18 further adjusts at least one of the intensity and a delay time of the ultrasound generated by each ultrasound transducer 32 in response to control of the controller 22.

The ultrasound probe 12, which extends in an up-and-down direction, is attached to the transducer housing 26 in such a manner that the ultrasound is transmitted and received at a position below the transducer housing 26 and above the therapeutic reference point P. In the present embodiment, the ultrasound probe 12 penetrates through a vertex part of the concave surface 30 of the transducer housing 26 in the up-and-down direction, and a transmission/reception unit 28 for transmitting and receiving the ultrasound faces downward.

Below the HIFU transducer unit 10, the coupling bag 25 is provided for matching acoustic impedance between the ultrasound transducers 32 and a patient, and between the ultrasound probe 12 and the patient. The coupling bag 24 may be a bag filled with a liquid such as water. The ultrasound probe 12 penetrates from above the coupling bag 24 into the inside of the coupling bag 24, and the transmission/reception unit 28 is positioned inside the coupling bag 24.

An ultrasound diagnostic apparatus may be used as the data acquisition unit 16. The data acquisition unit 16 executes the following processes in response to control of the controller 22. That is, the data acquisition unit 16 causes the ultrasound probe 12 to transmit the ultrasound, and to scan a beam (ultrasound beam) formed by the transmitted ultrasound.

A long axis direction and a short axis direction are defined for the transmission/reception unit 28 of the ultrasound probe 12, and the ultrasound beam is scanned on an observation surface 44 widening in the long axis direction and in a direction away from the transmission/reception unit 28. The ultrasound probe 12 is attached to the HIFU transducer unit 10 such that the observation surface 44 includes a central axis 40 which penetrates through the vertex part of the concave surface 30 of the transducer housing 26 in the up-and-down direction.

The data acquisition unit 16 causes the ultrasound probe 12 to receive a reflected ultrasound arriving from a direction to which the ultrasound beam is directed, and acquires a reception signal based on the reflected ultrasound received from each direction to which the ultrasound beam is directed. The data acquisition unit 16 produces ultrasound data based on the reception signal acquired at the observation surface 44, and outputs the ultrasound data to the controller 22. The ultrasound data may be, for example, data indicating a B mode image (tomographic image) acquired for the observation surface 44.

The ultrasound probe 12 is rotatable about the central axis 40 by the probe driver 14. The probe driver 14 rotates the ultrasound probe 12 about the central axis 40 with respect to the HIFU transducer unit 10. That is, the probe driver 14 rotates the ultrasound probe 12 about the central axis 40, to thereby rotate the observation surface 44 of the ultrasound probe 12 about the central axis 40, in response to control of the controller 22.

During therapy, the coupling bag 24, the ultrasound probe 12, and the HIFU transducer unit 10 are placed in such a manner that a lower part of the coupling bag 24 is in close contact with the patient. Before the therapeutic ultrasound is illuminated from the HIFU sonication unit 10 to the patient, the following positioning process is executed.

The transducer control unit 18 causes each ultrasound transducer 32 of the HIFU transducer unit 10 to transmit an ultrasound having a lower intensity than that during therapy. The probe driver 14 sets a rotational angle position (position represented by a rotational angle) of the ultrasound probe 12 so that the ultrasound beam is scanned on the observation surface 44 widening in a predetermined direction.

The data acquisition unit 16 causes the ultrasound probe 12 to scan the ultrasound beam on the observation surface 44 defined by the rotational angle position of the ultrasound probe 12, acquires the ultrasound data, and outputs the ultrasound data to the controller 22. The controller 22 causes a B mode image indicated by the ultrasound data and an image showing the therapeutic reference point P to be displayed in an overlapped manner on the display 20. The user who is the person executing the therapy refers to the image displayed on the display 20, and checks a difference between a position where the ultrasound transmitted from the HIFU transducer unit 10 is strengthened (focal point F) and the position of the therapeutic reference point P.

When the difference between the position of the focal point F and the position of the therapeutic reference point P falls outside of a tolerable range, the user changes positions or orientations of the coupling bag 24, the ultrasound probe 12, and the HIFU transducer unit 10. Alternatively, the user changes a contact state between the coupling bag 24 and the patient 34. After the user confirms that the position of the focal point F and the position of the therapeutic reference point P coincide or that the difference between the position of the focal point F and the position of the therapeutic reference point P is within the tolerable range, the user applies manipulation for therapy on the controller 22. The controller 22 controls the transducer control unit 18 in response to the manipulation by the user. The transducer control unit 18 causes each of the ultrasound transducers 32 to transmit the therapeutic ultrasound having intensity necessary for therapy in response to the control of the controller 22. In this manner, the biological tissue at the focal point F is cauterized, and therapy is applied.

In the therapy using the high intensity focused ultrasound, if a region having acoustic characteristics different from the surroundings exists in the tissue of the patient, there may be a case in which, due to refraction of the ultrasound emitted from the ultrasound transducer 32, the position of the focal point F is deviated from that in the case of uniform acoustic characteristics. As a consequence, a case may occur in which the focal point F of the ultrasound does not appear on the B mode image, and the positioning process becomes difficult.

Accordingly, in the HIFU sonication apparatus 100 according to the present embodiment, the following focal point adjustment process may be executed prior to the execution of the positioning process. With the focal point adjustment process, the focal point F moves to a position on the observation surface 44 of the ultrasound probe 12. Further, the focal point F may be set to move closer to the central axis 40, or may be set on the central axis 40.

The controller 22 controls the data acquisition unit 16 to cause the data acquisition unit 16 to acquire B mode image data as ultrasound data. The data acquisition unit 16 causes the ultrasound probe 12 to transmit the ultrasound, and scans the ultrasound beam on the observation surface 44. The data acquisition unit 16 causes the ultrasound probe 12 to receive a reflected ultrasound arriving from a direction to which the ultrasound beam is directed, and acquires a reception signal based on the ultrasound received reflected from each direction to which the ultrasound beam is directed. The data acquisition unit 16 produces ultrasound data based on the reception signal acquired on the observation surface 44, and outputs the ultrasound data to the controller 22.

The controller 22 also controls the transducer control unit 18 to cause each ultrasound transducer 32 of the HIFU transducer unit 10 to transmit an ultrasound having a lower intensity than that during therapy.

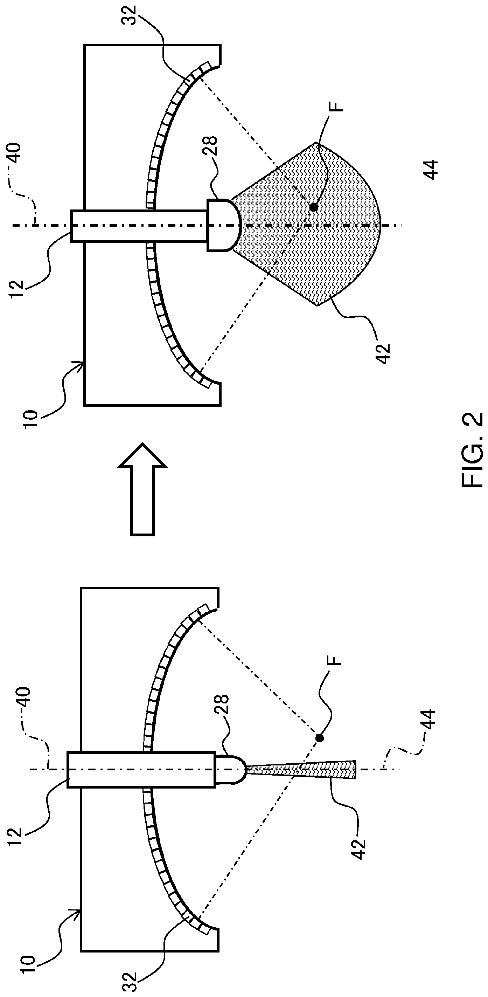

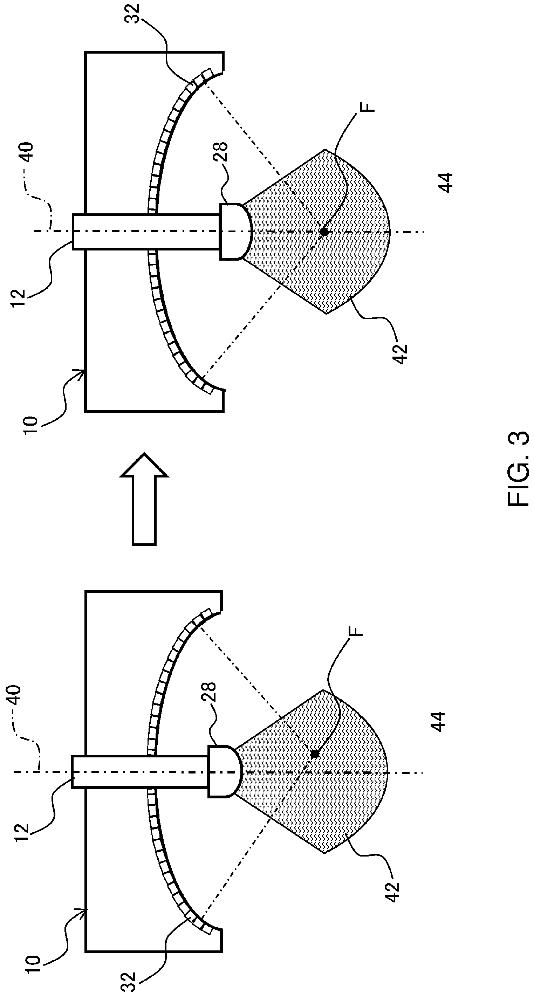

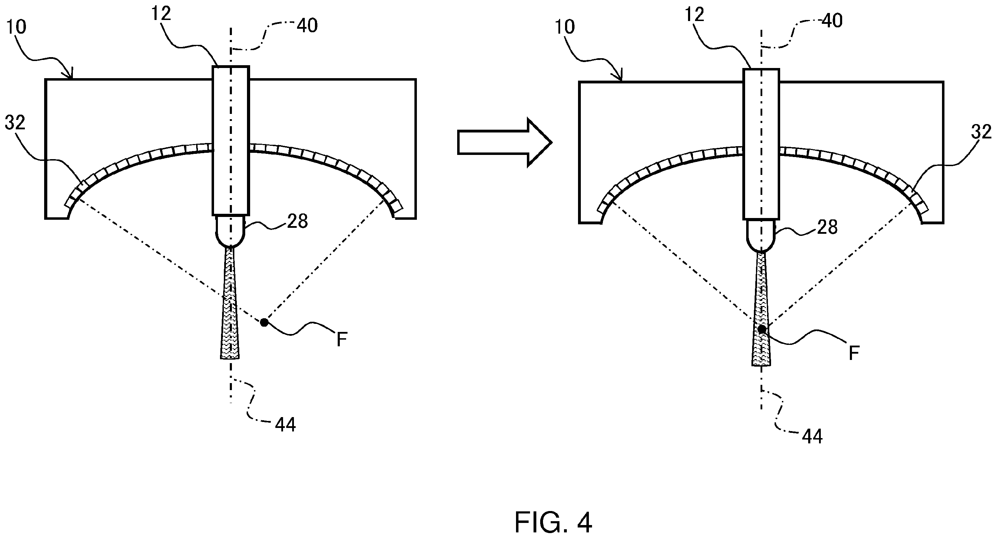

FIGs. 2 to 4 are diagrams for explaining the focal point adjustment process. At a left side of FIG. 2, a state is shown in which a short axis direction of the transmission/reception unit 28 points to the left and to the right. At a right side of FIG. 2, a state is shown in which a long axis direction of the transmission/reception unit 28 points to the left and to the right. FIG. 3 shows a state in which the long axis direction of the transmission/reception unit 28 points to the left and to the right. FIG. 4 shows a state in which the short axis direction of the transmission/reception unit 28 points to the left and to the right.

At the left side of FIG. 2, a state is shown in which a focal point F is present at a position distanced from the observation surface 44. Note that, in this figure, for clarification purpose, illustration of the coupling bag 24 is omitted. In FIGs. 3 and 4 to be described below also, the illustration of the coupling bag 24 is omitted. While the controller 22 controls the probe driver 14 to rotate the ultrasound probe 12 about the central axis 40, the controller 22 searches for a focal point F in the B mode image indicated by the ultrasound data which is output from the data acquisition unit 16. The probe driver 14 rotates the ultrasound probe 12 about the central axis 40 in a range of a minimum of 90°. The controller 22 controls the probe driver 14, to cause the ultrasound probe 12 to rest at a rotational angle position at which the focal point F is detected on the B mode image. At the right side of FIG. 2 and the left side of FIG. 3, an ultrasound beam scan region 42 and the focal point F when the focal point F is detected on the B mode image are schematically shown. Here, the ultrasound beam scan region 42 is a region in which the ultrasound beam is scanned, and the B mode image data is generated for this region.

The controller 22 controls the transducer control unit 18, to adjust the delay time of the ultrasound generated by each of the plurality of the ultrasound transducers 32, and to move the focal point F in the observation surface 44. That is, in response to the control of the controller 22, the transducer control unit 18 adjusts the delay time of the ultrasound generated by each of the plurality of ultrasound transducers 32, and moves the focal point F in the observation surface 44, so that the focal point F moves closer to the central axis 40 or the focal point F is positioned on the central axis. At the right side of FIG. 3, the ultrasound beam scan region 42 and the focal point F when the focal point F is positioned on the central axis 40 are schematically shown.

In the HIFU sonication apparatus 100, the positioning process may be executed in a state where the focal point F is present on the B mode image even if the focal point F is not positioned on the central axis 40, as shown at the left side of FIG. 3, or the positioning process may be executed in a state where the focal point F is positioned on the central axis 40, as shown at the right side of FIG. 3.

Alternatively, the focal point adjustment process may be executed by adjusting the delay time of the ultrasound emitted from each of the plurality of the ultrasound transducers 32 without rotating the ultrasound probe 12. At the left side of FIG. 4, a state is shown in which the focal point F is present at a position distanced from the observation surface 44. The controller 22 controls the transducer control unit 18, to adjust the delay time of the ultrasound generated by each of the plurality of ultrasound transducers 32, and to move the focal point F in a plane intersecting the observation surface 44, for example, a plane perpendicular to the observation surface 44. That is, in response to the control of the controller 22, the transducer control unit 18 adjusts the delay time of the ultrasound generated by each of the plurality of ultrasound transducers 32, to move the focal point F in the plane intersecting the observation surface 44, and to position the focal point F on the observation surface 44. At the right side of FIG. 4, the ultrasound beam scan region 42 and the focal point F when the focal point F is positioned on the observation surface 44 are schematically shown.

The state shown at the right side of FIG. 3 is identical to the state shown at the left side of FIG. 3. The controller 22 controls the transducer control unit 18, to adjust the delay time of the ultrasound generated by each of the plurality of ultrasound transducers 32, and to move the focal point F in the observation surface 44. That is, in response to the control of the controller 22, the transducer control unit 18 adjusts the delay time of the ultrasound generated by each of the plurality of ultrasound transducers 32, and moves the focal point F in the observation surface 44, so that the focal point F moves closer to the central axis 40 or the focal point F is positioned on the central axis 40. At the right side of FIG. 3, the ultrasound beam scan region 42 and the focal point F when the focal point F is positioned on the central axis 40 are schematically shown.

In the case in which the ultrasound probe 12 is not rotated also, in the HIFU sonication apparatus 100, the positioning process may be executed in a state in which the focal point F is present on the B mode image even when the focal point F is not positioned on the central axis 40, as shown at the left side of FIG. 3. Alternatively, the positioning process may be executed in a state in which the focal point F is positioned on the central axis 40, as shown at the right side of FIG. 3.

In this manner, in the HIFU sonication apparatus 100, the focal point adjustment process is executed by a method of adjusting the focal point including the following steps (i) to (iii):
(i) a step in which, during a period in which the plurality of the ultrasound transducers 32 are caused to transmit the ultrasounds, while the rotational angle position of the ultrasound probe 12 about the central axis 40 is changed, the focal point F of the ultrasounds emitted from the plurality of ultrasound transducers 32 is searched for in the B mode image indicated by the B mode image data;
(ii) a step in which the ultrasound probe 12 is caused to rest at the rotational angle position at which the focal point F is detected; and
(iii) a step in which, in a state in which the ultrasound probe 12 rests at the rotational angle position at which the focal point F is detected, the delay time of each of the ultrasounds emitted from the plurality of ultrasound transducers 32 is adjusted, so that the focal point F moves closer to the central axis 40 or the focal point F is positioned on the central axis 40.

In the focal point adjustment method, the step (iii) is not necessarily executed. The focal point adjustment method may be a method in which only the steps (i) and (ii) are executed.

Further, in the HIFU sonication apparatus, the focal point adjustment process may be executed by a method of adjusting the focal point including the following steps (a) and (b):
(a) a step in which, during a period in which the plurality of ultrasound transducers 32 are caused to transmit the ultrasounds, while the delay time of the ultrasound emitted from each of the plurality of ultrasound transducers 32 is adjusted, the focal point F of the ultrasounds emitted from the plurality of ultrasound transducers 32 is searched for in the B mode image indicated by the B mode image data; and
(b) a step in which, in a state in which each of the plurality of the ultrasound transducers 32 is caused to generate the ultrasound with the delay time at which the focal point F is detected, the delay time of the ultrasound emitted from each of the plurality of ultrasound transducers 32 is further adjusted, so that the focal point moves closer to the central axis 40, or the focal point F is positioned on the central axis 40.

In the focal point adjustment method, the step (b) is not necessarily executed. The focal point adjustment method may be a method in which only the step (a) is executed.

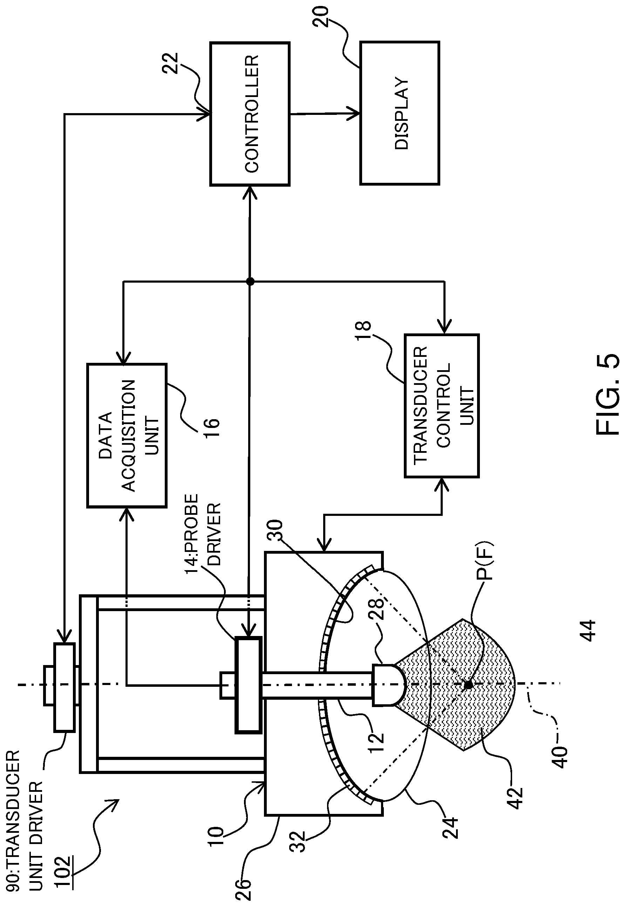

FIG. 5 shows a structure of a HIFU sonication apparatus 102 according to a second embodiment of the present invention. The HIFU sonication apparatus 102 is an apparatus in which a transducer unit driver 90 is added with respect to the HIFU sonication apparatus 100. In response to the control of the controller 22, the transducer unit driver 90 rotates the HIFU transducer unit 10 about the central axis 40 with respect to a reference coordinate system fixed on a bed on which the patient 34 lies down or the like. That is, the transducer unit driver 90 has a function as an ultrasound transducer driver which rotates the plurality of ultrasound transducers 32, placed along the concave surface 30, about the central axis 40.

In the present embodiment, the probe driver 14 maintains the ultrasound probe 12 in a rested state. Here, the rested state of the ultrasound probe 12 refers to a state in which the ultrasound probe 12 rests in the reference coordinate system. The transducer unit driver 90 rotates the HIFU transducer unit 10 in a rotational direction opposite from the rotational direction of the ultrasound probe 12 in the focal point adjustment process described above with reference to FIGs. 2 to 4, with regard to the first embodiment of the present invention.

The probe driver 14 rotates the ultrasound probe 12 about the central axis 40 with respect to the HIFU transducer unit 10. As such, by setting the rotational angle of the ultrasound probe 12 with respect to the HIFU transducer unit 12 to be the same angle but in the opposite direction as the rotational angle in the reference coordinate system of the HIFU transducer uni10, the ultrasound probe 12 can be set in the rested state in the reference coordinate system. With this configuration, the position of the focal point F on the B mode image can be adjusted without changing the B mode image displayed on the display 20.

In the HIFU sonication apparatus 102, a focal point adjustment process is executed by a method of adjusting the focal point including the following steps (α) to (γ):
(α) a step in which, during a period in which the plurality of ultrasound transducers 32 are caused to transmit the ultrasounds, while the rotational angle position of the HIFU transducer unit 10 about the central axis 40 is changed; that is, while the rotational angle positions of the plurality of ultrasound transducers 32 are changed, the focal point F of the ultrasounds emitted from the plurality of ultrasound transducers 32 is searched for in the B mode image indicated by the B mode image data;
(β) a step in which the HIFU transducer unit 10 is caused to rest at the rotational angle position at which the focal point F is detected; and
(γ) a step in which, in a state in which the HIFU transducer unit 10 rests at the rotational angle position at which the focal point F is detected, the delay time of each of the ultrasounds emitted from the plurality of ultrasound transducers 32 is adjusted, so that the focal point moves closer to the central axis 40 or the focal point F is positioned on the central axis 40.

In the focal point adjustment method, the step (γ) is not necessarily executed. The focal point adjustment method may be a method in which only the steps (α) and (β) are executed.

According to the focal point adjustment process described above, the focal point F can be positioned on the observation surface 44. Therefore, even in the case in which the focal point F does not appear in the B mode image due to refraction, the positioning process can be executed by positioning the focal point F on the observation surface 44. In addition, in the focal point adjustment process, the focal point F may be moved closer to the central axis 40 or the focal point F may be set on the central axis 40. In this case, the focal point F is positioned below the ultrasound probe 12, and an image of the focal point F is positioned at an easily viewable position in the B mode image. Therefore, the operation of the positioning process can be facilitated.

### [Configurations of Present Invention]

### Configuration 1:

A HIFU sonication apparatus comprising:
a transducer control unit configured to control a plurality of ultrasound transducers placed along a concave surface;
a probe driver that rotates an ultrasound probe, placed on a central axis of the concave surface, about the central axis;
a data acquisition unit that acquires B mode image data by the ultrasound probe; and
a controller configured to control the transducer control unit, the probe driver, and the data acquisition unit, wherein
the controller is configured to execute:
   a process in which, during a period in which the plurality of ultrasound transducers are caused to transmit ultrasounds, while a rotational angle position of the ultrasound probe about the central axis is changed, a focal point of the ultrasounds emitted from the plurality of ultrasound transducers is searched for in a B mode image indicated by the B mode image data; and
   a process in which the ultrasound probe is caused to rest at the rotational angle position at which the focal point is detected.

### Configuration 2:

The HIFU sonication apparatus according to configuration 1, wherein
the controller is configured to, in a state in which the ultrasound probe rests at the rotational angle position at which the focal point is detected, adjust a delay time of the ultrasound emitted from each of the plurality of ultrasound transducers, so that the focal point moves closer to the central axis or the focal point is positioned on the central axis.

### Configuration 3:

A HIFU sonication apparatus comprising:
a transducer control unit configured to control a plurality of ultrasound transducers placed along a concave surface;
an ultrasound transducer driver that rotates the plurality of ultrasound transducers about a central axis of the concave surface;
a data acquisition unit that acquires B mode image data by an ultrasound probe placed on the central axis of the concave surface; and
a controller configured to control the transducer control unit, the ultrasound transducer driver, and the data acquisition unit, wherein
the controller is configured to execute:
   a process in which, during a period in which the plurality of ultrasound transducers are caused to transmit ultrasounds, while rotational angle positions of the plurality of ultrasound transducers about the central axis are changed, a focal point of the ultrasounds emitted from the plurality of ultrasound transducers is searched for in a B mode image indicated by the B mode image data; and
   a process in which the plurality of ultrasound transducers are caused to rest at the rotational angle positions at which the focal point is detected.

### Configuration 4:

The HIFU sonication apparatus according to configuration 3, wherein
the controller is configured to, in a state in which the plurality of ultrasound transducers rest at the rotational angle positions at which the focal point is detected, adjust a delay time of the ultrasound emitted from each of the plurality of ultrasound transducers, so that the focal point moves closer to the central axis or the focal point is positioned on the central axis.

### Configuration 5:

A HIFU sonication apparatus comprising:
a transducer control unit configured to control a plurality of ultrasound transducers placed along a concave surface;
a data acquisition unit that acquires B mode image data by an ultrasound probe placed on a central axis of the concave surface; and
a controller configured to control the transducer control unit and the data acquisition unit, wherein
the controller is configured to execute a process in which, during a period in which the plurality of ultrasound transducers are caused to transmit ultrasounds, while a delay time of the ultrasound emitted from each of the plurality of ultrasound transducers is adjusted, a focal point of the ultrasounds emitted from the plurality of ultrasound transducers is searched for in a B mode image indicated by the B mode image data.

### Configuration 6:

The HIFU sonication apparatus according to configuration 5, wherein
in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.

### Configuration 7:

A method of adjusting a focal point of a HIFU sonication apparatus, the HIFU sonication apparatus comprising:
a plurality of ultrasound transducers placed along a concave surface;
an ultrasound probe placed on a central axis of the concave surface, in a manner to be rotatable about the central axis; and
a data acquisition unit which acquires B mode image data by the ultrasound probe,
the method comprising:
   a step in which, during a period in which the plurality of ultrasound transducers are caused to transmit ultrasounds, while a rotational angle position of the ultrasound probe about the central axis is changed, a focal point of the ultrasounds emitted from the plurality of ultrasound transducers is searched for in a B mode image indicated by the B mode image data; and
   a step in which the ultrasound probe is caused to rest at the rotational angle position at which the focal point is detected.

### Configuration 8:

The method of adjusting the focal point according to configuration 7, further comprising:
a step in which, in a state in which the ultrasound probe rests at the rotational angle position at which the focal point is detected, a delay time of each of the ultrasounds emitted from the plurality of ultrasound transducers is adjusted, so that the focal point moves closer to the central axis or the focal point is positioned on the central axis.

### Configuration 9:

A method of adjusting a focal point of a HIFU sonication apparatus, the HIFU sonication apparatus comprising:
a plurality of ultrasound transducers placed along a concave surface, and placed in a manner to be rotatable about a central axis of the concave surface;
an ultrasound probe placed on the central axis of the concave surface; and
a data acquisition unit which acquires B mode image data by the ultrasound probe,
the method comprising:
   a step in which, during a period in which the plurality of ultrasound transducers are caused to transmit ultrasounds, while rotational angle positions of the plurality of ultrasound transducers about the central axis are changed, a focal point of the ultrasounds emitted from the plurality of ultrasound transducers is searched for in a B mode image indicated by the B mode image data; and
   a step in which the plurality of ultrasound transducers are caused to rest at the rotational angle positions at which the focal point is detected.

### Configuration 10:

The method of adjusting the focal point according to configuration 9, further comprising:
a step in which, in a state in which the plurality of ultrasound transducers rest at the rotational angle positions at which the focal point is detected, a delay time of each of the ultrasounds emitted from the plurality of ultrasound transducers is adjusted, so that the focal point moves closer to the central axis or the focal point is positioned on the central axis.

### Configuration 11:

A method of adjusting a focal point of a HIFU sonication apparatus, the HIFU sonication apparatus comprising:
a plurality of ultrasound transducers placed along a concave surface; and
a data acquisition unit which acquires B mode image data by an ultrasound probe placed on a central axis of the concave surface,
the method comprising:
   a step in which, during a period in which the plurality of ultrasound transducers are caused to transmit ultrasounds, while a delay time of the ultrasound emitted from each of the plurality of ultrasound transducers is adjusted, a focal point of the ultrasounds emitted from the plurality of ultrasound transducers is searched for in a B mode image indicated by the B mode image data.

### Configuration 12:

The method of adjusting the focal point according to claim 11, further comprising:
a step in which, in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.

### REFERENCE SIGNS LIST

10 HIFU transducer unit, 12 ultrasound probe, 14 probe driver, 16 data acquisition unit, 18 transducer control unit, 20 display, 22 controller, 24 coupling bag, 26 transducer housing, 28 transmission/reception unit, 30 concave surface, 32 ultrasound transducer, 40 central axis, 42 ultrasound beam scan region, 44 observation surface, 90 transducer unit driver, 100, 102 HIFU sonication apparatus.

## Claims

1. A HIFU sonication apparatus comprising:
a transducer control unit configured to control a plurality of ultrasound transducers placed along a concave surface;
a probe driver that rotates an ultrasound probe, placed on a central axis of the concave surface, about the central axis;
a data acquisition unit that acquires B mode image data by the ultrasound probe; and
a controller configured to control the transducer control unit, the probe driver, and the data acquisition unit, wherein
the controller is configured to execute:
a process in which, during a period in which the plurality of ultrasound transducers are caused to transmit ultrasounds, while a rotational angle position of the ultrasound probe about the central axis is changed, a focal point, of the ultrasounds emitted from the plurality of ultrasound transducers and which appears as a magnitude of brightness in a B mode image indicated by the B mode image data, is searched for based on the B mode image data; and
a process in which the ultrasound probe is caused to rest at the rotational angle position at which the focal point is detected.

2. The HIFU sonication apparatus according to claim 1, wherein
the controller is configured to, in a state in which the ultrasound probe rests at the rotational angle position at which the focal point is detected, adjust a delay time of the ultrasound emitted from each of the plurality of ultrasound transducers, so that the focal point moves closer to the central axis or the focal point is positioned on the central axis.

3. A HIFU sonication apparatus comprising:
a transducer control unit configured to control a plurality of ultrasound transducers placed along a concave surface;
an ultrasound transducer driver that rotates the plurality of ultrasound transducers about a central axis of the concave surface;
a data acquisition unit that acquires B mode image data by an ultrasound probe placed on the central axis of the concave surface; and
a controller configured to control the transducer control unit, the ultrasound transducer driver, and the data acquisition unit, wherein
the controller is configured to execute:
a process in which, during a period in which the plurality of ultrasound transducers are caused to transmit ultrasounds, while rotational angle positions of the plurality of ultrasound transducers about the central axis are changed, a focal point, of the ultrasounds emitted from the plurality of ultrasound transducers and which appears as a magnitude of brightness in a B mode image indicated by the B mode image data, is searched for based on the B mode image data; and
a process in which the plurality of ultrasound transducers are caused to rest at the rotational angle positions at which the focal point is detected.

4. The HIFU sonication apparatus according to claim 3, wherein
the controller is configured to, in a state in which the plurality of ultrasound transducers rest at the rotational angle positions at which the focal point is detected, adjust a delay time of the ultrasound emitted from each of the plurality of ultrasound transducers, so that the focal point moves closer to the central axis or the focal point is positioned on the central axis.

5. A method of adjusting a focal point of a HIFU sonication apparatus, wherein
the HIFU sonication apparatus comprises:
a plurality of ultrasound transducers placed along a concave surface;
an ultrasound probe placed on a central axis of the concave surface, in a manner to be rotatable about the central axis; and
a data acquisition unit which acquires B mode image data by the ultrasound probe,
the HIFU sonication apparatus further comprises:
a transducer control unit configured to control the plurality of ultrasound transducers; and
a probe driver which rotates the ultrasound probe about the central axis, and
a controller, configured to control the transducer control unit, the probe driver, and the data acquisition unit, is configured to execute:
a step in which, during a period in which the plurality of ultrasound transducers are caused to transmit ultrasounds, while a rotational angle position of the ultrasound probe about the central axis is changed, a focal point, of the ultrasounds emitted from the plurality of ultrasound transducers and which appears as a magnitude of brightness in a B mode image indicated by the B mode image data, is searched for based on the B mode image data; and
a step in which the ultrasound probe is caused to rest at the rotational angle position at which the focal point is detected.

6. The method of adjusting the focal point according to claim 5, wherein
the controller is configured to execute a step in which, in a state in which the ultrasound probe rests at the rotational angle position at which the focal point is detected, a delay time of each of the ultrasounds emitted from the plurality of ultrasound transducers is adjusted, so that the focal point moves closer to the central axis or the focal point is positioned on the central axis.

7. A method of adjusting a focal point of a HIFU sonication apparatus, wherein
the HIFU sonication apparatus comprises:
a plurality of ultrasound transducers placed along a concave surface, and placed in a manner to be rotatable about a central axis of the concave surface;
an ultrasound probe placed on the central axis of the concave surface; and
a data acquisition unit which acquires B mode image data by the ultrasound probe,
the HIFU sonication apparatus further comprises:
a transducer control unit configured to control the plurality of ultrasound transducers; and
a probe driver which rotates the ultrasound probe about the central axis, and
a controller, configured to control the transducer control unit, the probe driver, and the data acquisition unit, is configured to execute:
a step in which, during a period in which the plurality of ultrasound transducers are caused to transmit ultrasounds, while rotational angle positions of the plurality of ultrasound transducers about the central axis are changed, a focal point, of the ultrasounds emitted from the plurality of ultrasound transducers and which appears as a magnitude of brightness in a B mode image indicated by the B mode image data, is searched for based on the B mode image data; and
a step in which the plurality of ultrasound transducers are caused to rest at the rotational angle positions at which the focal point is detected.

8. The method of adjusting the focal point according to claim 7, wherein
the controller is configured to execute a step in which, in a state in which the plurality of ultrasound transducers rest at the rotational angle positions at which the focal point is detected, a delay time of each of the ultrasounds emitted from the plurality of ultrasound transducers is adjusted, so that the focal point moves closer to the central axis or the focal point is positioned on the central axis.

9. A HIFU sonication apparatus comprising:
a transducer control unit configured to control a plurality of ultrasound transducers placed along a concave surface;
a data acquisition unit that acquires B mode image data by an ultrasound probe placed on a central axis of the concave surface; and
a controller configured to control the transducer control unit and the data acquisition unit, wherein
the controller is configured to execute a process in which, during a period in which the plurality of ultrasound transducers are caused to transmit ultrasounds, while a delay time of the ultrasound emitted from each of the plurality of ultrasound transducers is adjusted, a focal point, of the ultrasounds emitted from the plurality of ultrasound transducers and which appears as a magnitude of brightness in a B mode image indicated by the B mode image data, is searched for based on the B mode image data.

10. The HIFU sonication apparatus according to claim 9, wherein
in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.

11. A method of adjusting a focal point of a HIFU sonication apparatus, wherein
the HIFU sonication apparatus comprises:
a plurality of ultrasound transducers placed along a concave surface; and
a data acquisition unit which acquires B mode image data by an ultrasound probe placed on a central axis of the concave surface,
the HIFU sonication apparatus further comprises a transducer control unit configured to control the plurality of ultrasound transducers, and
a controller, configured to control the transducer control unit and the data acquisition unit, is configured to execute a step in which, during a period in which the plurality of ultrasound transducers are caused to transmit ultrasounds, while a delay time of the ultrasound emitted from each of the plurality of ultrasound transducers is adjusted, a focal point, of the ultrasounds emitted from the plurality of ultrasound transducers and which appears as a magnitude of brightness in a B mode image indicated by the B mode image data, is searched for based on the B mode image data.

12. The method of adjusting the focal point according to claim 11, wherein
the controller is further configured to execute a step in which, in a state in which each of the plurality of ultrasound transducers is caused to generate the ultrasound with the delay time at which the focal point is detected, the delay time of the ultrasound emitted from each of the plurality of ultrasound transducers is further adjusted, so that the focal point moves closer to the central axis or the focal point is positioned on the central axis.
